# EUROPEAN PATENT APPLICATION

(11) **EP 3 263 073 A1**
(43) Date of publication of application: **03.01.2018**
(21) Application number: 16755125.8
(22) Date of filing: 28.01.2016
(51) Int. Cl.: A61F 2/89

(54) **STENT AND METHOD FOR MANUFACTURING STENT**

(30) Priority: 27.02.2015 JP 2015039349
(71) Applicant: Terumo Kabushiki Kaisha, Tokyo 151-0072 (JP)
(72) Inventor: YAMAMOTO, Toshihiro, Ashigarakami-gun Kanagawa 259-0151 (JP)
(74) Representative: Casalonga
(86) International application number: PCT/JP2016/052543
(87) International publication number: WO 2016/136375

(57) **Abstract**

[Problem] In order to provide a stent and a method of manufacturing a stent, capable of more improving fracture resistance.

[Means for Resolution] A stent 100 has metal portions 110 that shape a tubular outer periphery provided with a gap 111 and a polymer portion 120 that connects the metal portions to each other in the gap. The polymer portion has a curved portion 121 that is curved to be concave toward an outer side from an inner side in a radial direction of the tubular outer periphery.

## Description

### [Technical Field]

The present invention relates to a stent and a method of manufacturing a stent.

### [Background Art]

In recent years, a technique of forming a stent using metal and polymer has been proposed. For example, Patent Document 1 discloses a stent in which a helical outer peripheral portion is formed of metal, and a connection portion that connects the helical metal portions to each other is formed of polymer. By forming the stent using metal and polymer, it is possible to achieve both of two contradictory properties of a strength and flexibility.

### [Citation List]

### [Patent Document]

[Patent Document 1] Pamphlet of International Publication No. 2007/079363

### [Summary of the Invention]

### [Problems to be solved by the Invention]

However, when a tensile force is applied to an interface between metal and polymer such that the metal and the polymer are separated from each other, fracture resistance is reduced compared to a case where the entire stent is integrally formed of only metal or polymer. In this regard, it is conceived that the fracture resistance of the stent can be improved by hindering a strong force from being applied to the interface between the metal and the polymer.

The invention has been made in view of the aforementioned problems, and an object thereof is to provide a stent and a method of manufacturing a stent, capable of more improving fracture resistance.

### [Means for Solving the Problems]

A stent of the invention for attaining the above-described object includes: metal portions that shape a tubular outer periphery provided with a gap; and a polymer portion that connects the metal portions to each other in the gap. The polymer portion has a curved portion that is curved to be concave toward an outer side from an inner side in a radial direction of the tubular outer periphery.

A method of manufacturing a stent of the invention for attaining the above-described object includes: a polymer placement process for placing polymer toward a gap formed by metal portions that shape a tubular outer periphery of a stent; and a heating process for heating the polymer after the polymer placement process. In the heating process, the polymer is molten by the heating and flows to the gap, so that a curved portion that is curved to be concave toward an outer side from an inner side in a radial direction of the tubular outer periphery is formed in the polymer.

### [Advantage of the Invention]

According to the invention, the polymer portion which is relatively easily stretchable compared to the metal portions is thinned by forming the curved portion. Therefore, the polymer portion becomes more easily stretchable. For this reason, when a tensile force is applied to separate the metal portions and the polymer portion from each other, a strong force is not easily applied to an interface between the metal portions and the polymer portion by virtue of the stretch of the polymer portion. Therefore, it is possible to more improve the fracture resistance.

### [Brief Description of the Drawings]

Fig. 1 is a diagram illustrating a stent according to an embodiment.
Fig. 2 is a cross-sectional view taken along the line 2-2 of Fig. 1.
Fig. 3 is a diagram illustrating an overview of a method of manufacturing a stent according to an embodiment.
Fig. 4 is a cross-sectional view illustrating a modification of a polymer portion.
Fig. 5 is a cross-sectional view illustrating another modification of the polymer portion.
Fig. 6 is a cross-sectional view illustrating a modification in which a polymer layer is formed along with the polymer portion.
Fig. 7 is a cross-sectional view illustrating another modification in which a polymer layer is formed along with the polymer portion.
Fig. 8 is a cross-sectional view illustrating further another modification of the polymer portion.
Fig. 9 is an enlarged view illustrating a main portion according to a modification in which a connection portion is provided along with the polymer portion.
Fig. 10 is a cross-sectional view taken along the line 10-10 of Fig. 9.

### [Mode for Carrying Out the Invention]

Embodiments of the invention will now be described with reference to the accompanying drawings. Note that dimensions or scales of the drawings may be exaggerated or may be different from reality for convenient description purposes.

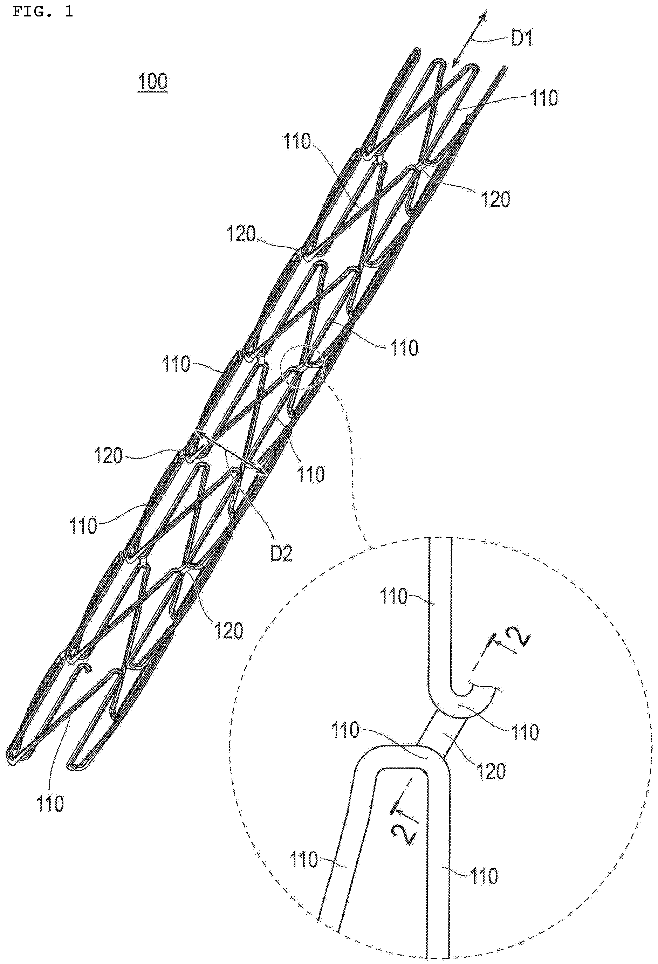

As illustrated in Fig. 1, a stent 100 according to an embodiment has a strut 110 (metal portion) and a polymer portion 120.

The stent 100 is used in a lumen such as a blood vessel, a bile duct, a trachea, an esophagus, a gastrointestinal tract, and a urethra in a living body. The stent 100 treats stenosis or obstruction by forcibly widening a bore of the lumen. The stent 100 may be a balloon-expandable stent which is expanded by a balloon or a self-expandable stent which expands by its own expanding function.

The strut 110 is a linear component formed of metal. The strut 110 shapes a tubular outer periphery provided with gaps in the stent 100.

For example, the strut 110 extends helically around the axial direction D1 of the stent 100 while being meandered in a wave shape or forms an endless annular body while being meandered in a wave shape. In addition, the struts 110 are connected coaxially along the axial direction D1 of the stent 100 so as to shape an outer periphery of the stent 100. Alternatively, helical portions extending around the axial direction D1 and endless annular portions may be connected coaxially in the axial direction D1 of the stent 100 so as to shape an outer periphery of the stent 100. The shape of the strut 110 is not particularly limited. The axial direction D1 of the stent 100 is perpendicular to a radial direction D2 of the tubular outer periphery of the stent 100 (hereinafter, simply referred to as a radial direction D2 of the stent 100).

The metal of the strut 110 may include, for example, stainless steel, tantalum, tantalum alloy, titanium, titanium alloy, nickel titanium alloy, tantalum titanium alloy, nickel aluminum alloy, Inconel, gold, platinum, iridium, tungsten, tungsten alloy, cobalt-based alloy such as cobalt chromium alloy, magnesium, zirconium, niobium, zinc, or silicon, but not particularly limited thereto. The metal of the strut 110 may be either biodegradable metal or non-biodegradable metal.

The polymer portion 120 is provided in a gap formed by the strut 110 to connect the struts 110 to each other. There is no particular limitation to where the polymer portion 120 is provided in the gap of the outer periphery of the stent 100 as long as the polymer portion 120 connects metal members of the stent 100 to each other.

The polymer portion 120 is formed of, for example, biodegradable polymer. The biodegradable polymer includes, for example, a biodegradable synthetic polymer material polylactic acid, polyglycolic acid, lactic acid-glycolic acid copolymer, polycaprolactone, lactic acid-caprolactone copolymer, glycolic acid-caprolactone copolymer, poly-γ-glutamin acid, a natural biodegradable polymer material such as cellulose or collagen, or the like. The polymer portion 120 may also be formed of non-biodegradable polymer.

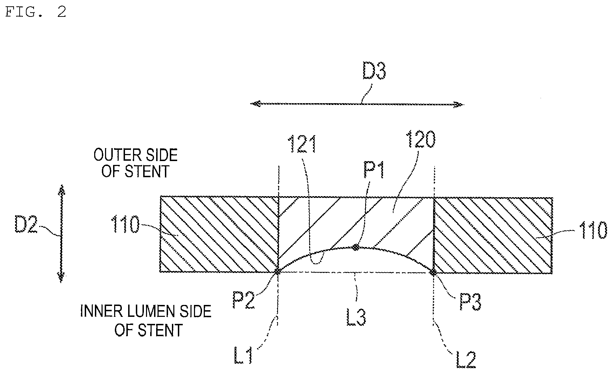

As illustrated in Fig. 2, the polymer portion 120 has a curved portion 121 in an inner lumen side (inner side) of the stent 100. The curved portion 121 is curved to be concave from the inner lumen side toward the outer side in the radial direction D2 of the stent 100. The curved portion 121 has a curvature different from a circumferential curvature of the stent 100.

In the cross section of Fig. 2 (a cross section taken along a separation direction of the metal portions), a peak P1 where the curved portion 121 is deepest is positioned in a center between two boundary lines L1 and L2 formed between the polymer portion 120 and two adjacent struts 110. In addition, the peak P1 is deviated from a line L3 obtained by straightly connecting two cross points P2 and P3 between the boundary lines L1 and L2 and the contour line of the curved portion 121. That is, the peak P1 exists in a different position that does not cross the line L3 obtained by straightly connecting the two cross points P2 and P3 between the boundary lines L1 and L2 and the contour line of the curved portion 121.

The cross section of Fig. 2 is a cross section obtained by cutting away the polymer portion 120 and the strut 110 on a plane defined by allowing the separation direction D3 of the struts 110 and the radial direction D2 of the stent 100 perpendicular thereto to cross each other.

Next, a method of manufacturing the stent 100 will be described.

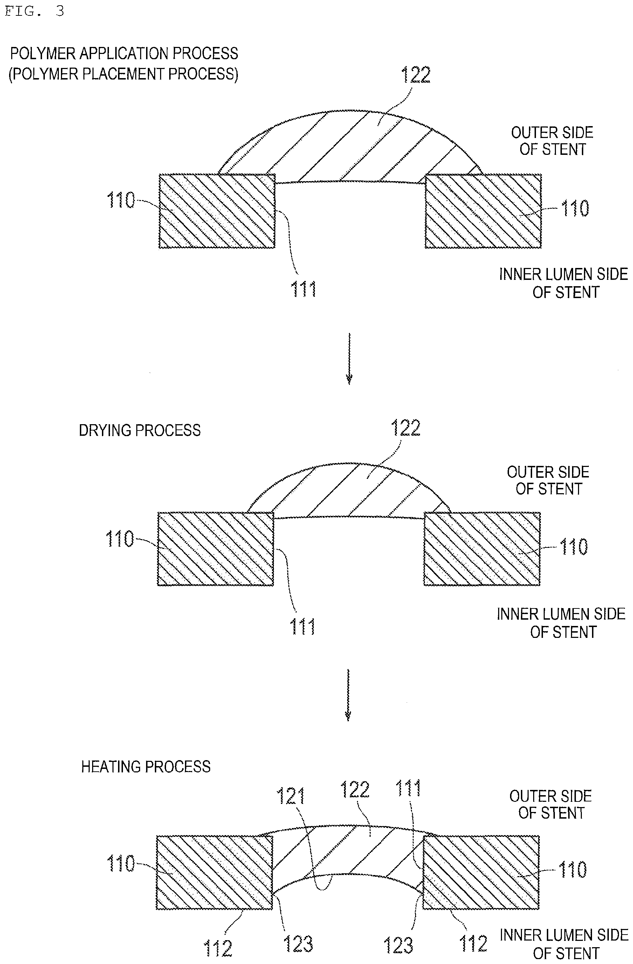

As illustrated in Fig. 3, the method of manufacturing the stent 100 has a polymer application process, a drying process, and a heating process. Before the polymer application process, the strut 110 having a predetermined shape is prepared.

In the polymer application process, a polymer solution 122 is applied toward the gap 111 formed by the strut 110. The polymer solution 122 is applied toward the gap 111 from the outer side of the stent. The polymer solution 122 is applied using an application device such as a micro-syringe (not illustrated).

The polymer solution 122 is obtained by dissolving polymer of the polymer portion 120 in a solvent. The solvent includes, for example, an organic solvent such as methanol, ethanol, dioxane, tetrahydrofuran, dimethylformamide, acetonitrile, dimethylsulfoxide, or acetone, and the like.

In the drying process after the polymer application process, the polymer solution 122 is dried, and the solvent is evaporated. The drying of the polymer solution 122 may include, for example, natural drying. Alternatively, the drying may also include heated drying by heating the polymer solution 122 without a particular limitation thereto. The drying reduces a volume of the polymer solution 122 and increase viscosity of the polymer solution 122.

After the drying process, the dried polymer solution 122 is heated in the heating process to further evaporate the solvent and melt the polymer contained in the solution. In the heating process, for example, the polymer solution 122 is heated inside a vacuum furnace together with the strut 110. Here, the heating temperature of the polymer solution 122 may be set to a temperature at which the polymer has sufficient fluidity. The temperature may vary depending on a type of the polymer and may be set to, for example, 35°C to 300°C without a particular limitation.

The fluidity of the polymer solution 122 heated through the heating process increases, so that the polymer solution 122 flows into the gap 111 by virtue of a capillary phenomenon. As a result, the curved portion 121 is formed on a surface of the polymer solution 122 in the inner lumen side of the stent. According to this embodiment, the polymer solution 122 is filled in the gap 111 until end portions 123 of the curved portion 121 reach the same position as the stent lumen-side surface 112 of the strut 110. In this manner, since the polymer solution 122 is filled in the inside of the gap 111 as much as possible, a contact area between the polymer solution 122 and the strut 110, and further, a contact area between the polymer portion 120 and the strut 110 increases. Therefore, it is possible to improve fracture resistance on such an interface. In addition, since more polymer solution 122 is filled, a volume of the polymer portion 120 increases. Therefore, it is possible to improve a strength of the polymer portion 120 of itself. After the polymer solution 122 is filled in the gap 111, the polymer solution 122 is solidified to form the polymer portion 120.

Next, functional effects of the present invention will be described.

According to this embodiment, compared to the strut 110 formed of metal, the relatively easily stretchable polymer portion 120 is thinned by forming the curved portion 121. Therefore, the strut 110 becomes more easily stretchable. For this reason, for example, when a tensile force is applied so as to separate the strut 110 and the polymer portion 120 from each other by expanding the stent 100, it is possible to prevent a strong force from being easily applied to an interface between the strut 110 and the polymer portion 120 by virtue of the stretch of the polymer portion 120 and thus improve fracture resistance.

In this embodiment, the peak P1 of the curved portion 121 is positioned in the center between two boundary lines L1 and L2 formed between the polymer portion 120 and the adjacent struts 110. In this configuration, the polymer portion 120 easily stretches evenly between one side and the other side of the two adjacent struts 110, and a force is substantially uniformly applied to two interfaces between the polymer portion 120 and both the adjacent struts 110. For this reason, it is possible to prevent fracture resistance from being lowered by biasedly applying a stronger force to any one of the two interfaces.

Unlike this embodiment, a polymer portion having a contour line, for example, as indicated by the line L3 of Fig. 2 is not thinned by the curved portion 121 compared to the polymer portion 120 of this embodiment. Therefore, it is not easily stretched relatively to the polymer portion 120.

Meanwhile, according to this embodiment, the peak P1 of the curved portion 121 is deviated to the outer side of the stent from the line L3. As a result, the polymer portion 120 is thinned and becomes easily stretchable. Therefore, a strong force is not easily applied to an interface between the strut 110 and the polymer portion 120. In addition, it is possible to improve fracture resistance.

The invention is not limited to the aforementioned embodiments, and may be modified in various forms within the scope of the claims.

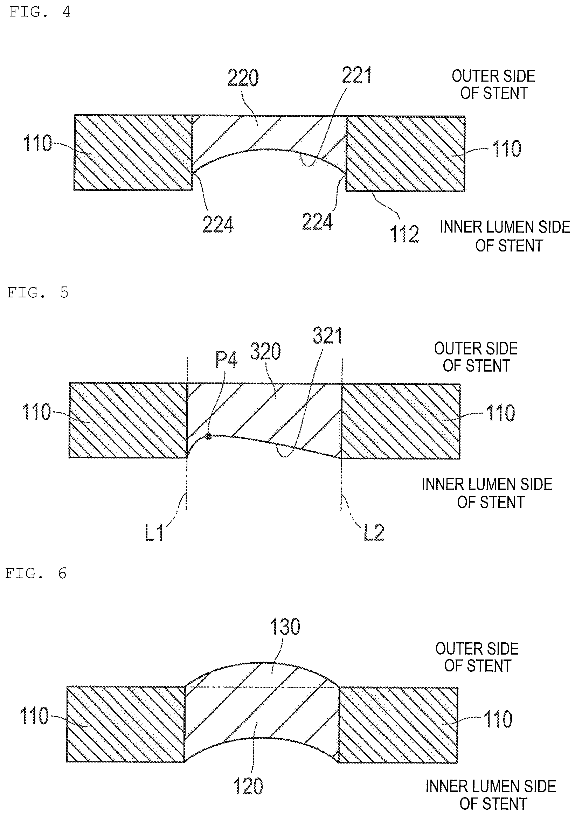

For example, as the polymer portion 220 illustrated in Fig. 4, the end portions 224 of the curved portion 221 may be positioned to be closer to the outer side of the stent than the stent lumen-side surface 112 of the strut 110.

The invention includes a polymer portion 320 illustrated in Fig. 5. In the polymer portion 320, the peak P4 of the curved portion 321 is deviated from the center between the boundary lines L1 and L2. That is, the peak P4 exists in a position different from the center between the boundary lines L1 and L2. In this case, the stretch of the polymer portion 320 is different between one side and the other side of the both adjacent struts 110, so that forces having different strengths are applied to two interfaces between the polymer portion 320 and both the adjacent struts 110. For this reason, a relatively stronger force is intentionally applied to any one of the two interfaces by deviating the peak P4 from the center. As a result, even when a fracture occurs, it is possible to control where the fracture occurs out of the two interfaces.

In addition, as illustrated in Fig. 6, a polymer layer 130 may be formed outward of the polymer portion 120 of the stent. The polymer layer 130 is formed to match a position of the polymer portion 120 and is interspersed on the outer periphery of the stent 100. Since the polymer portion 120 is reinforced by the polymer layer 130, it is possible to improve a strength of the polymer portion 120 of itself.

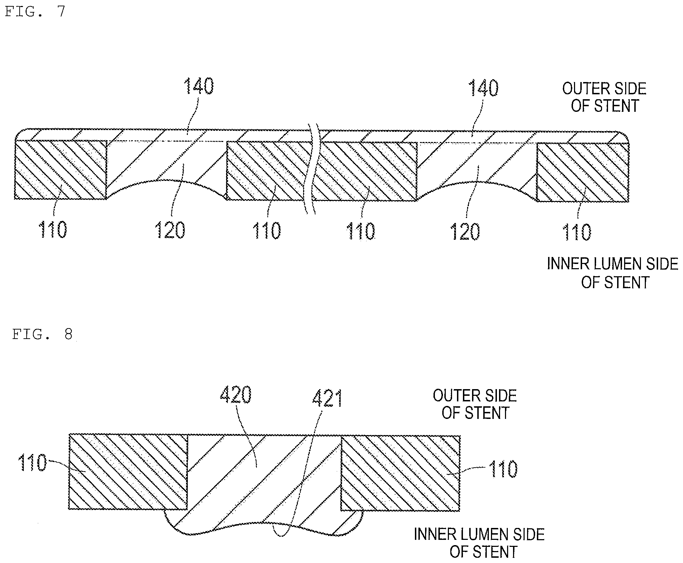

In addition, as illustrated in Fig. 7, a polymer layer 140 may be formed to continuously extend along the surface of the strut 110. The polymer layer 140 connects one of the polymer portions 120 and at least one of the other polymer portions 120. Since the polymer layer 140 reinforces the strut 110 and the polymer portion 120 across a wide range, it is possible to further improve the strength of the stent 100. The polymer layer 140 is preferably formed on the entire outer surface of the strut 110. Alternatively, without being limited thereto, the polymer layer 140 may be partially formed to extend in a part of the outer surface of the strut 110. Furthermore, the polymer layer 140 may be formed inward of the stent 100.

The polymer layers 130 and 140 are, for example, drug layers, but not limited thereto. In addition, the polymer layers 130 and 140 may be formed of the same material as that of the polymer portion 120 or a material different from that of the polymer portion 120. The polymer layers 130 and 140 are formed, for example, by further applying the polymer solution after formation of the polymer portion 120 and heating it for drying. A primer layer may also be formed before formation of the polymer layers 130 and 140.

As illustrated in Fig. 8, the invention also includes a polymer portion 420 protruding toward the stent inner lumen side with respect to the strut 110. As a result, a volume of the polymer portion 420 increases. Therefore, it is possible to improve a strength of the polymer portion 420 of itself.

Note that, in the polymer application process (polymer placement process) of the aforementioned embodiment, the polymer is placed in the gap 111 by applying the polymer solution 122. However, the invention is not limited thereto. For example, the polymer may be placed in the gap 111 by overlaying a solid sheet formed of polymer on the gap 111. In this case, the sheet is heated through a heating process and is molten, so that the molten polymer flows into the gap 111.

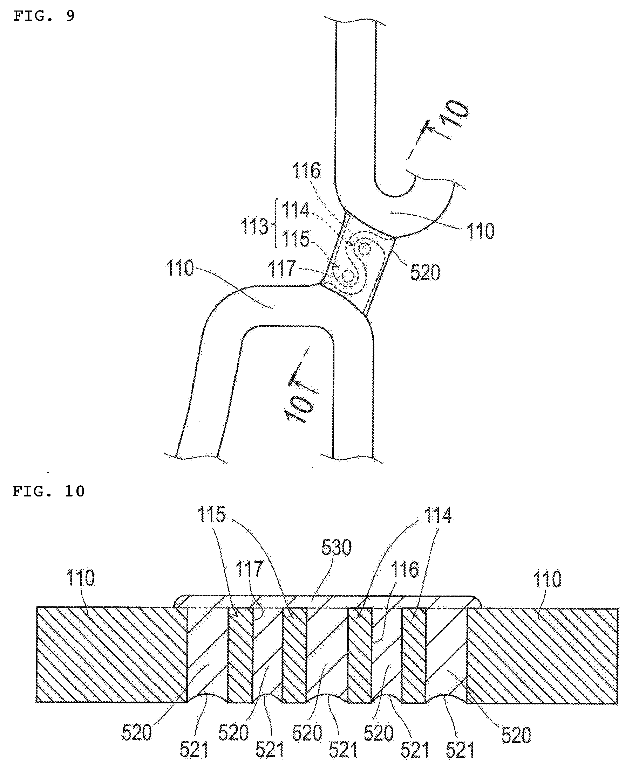

As illustrated in Fig. 9, the invention includes a stent provided with a connection portion 113 along with the polymer portion 520. The polymer portion 520 and the connection portion 113 connect the struts 110 to each other.

The connection portion 113 includes a first connection portion 114 and a second connection portion 115. The first connection portion 114 is formed integrally with one of the two struts 110 connected to each other, and the second connection portion 115 is formed integrally with the other strut 110. The first and second connection portions 114 and 115 are formed of the same metal as that of the strut 110. The first and second connection portions 114 and 115 are configured to form a gap having a substantially S-shape therebetween. The first and second connection portions 114 and 115 may partially make contact with each other.

The first connection portion 114 is provided with a first through-hole 116, and the second connection portion 115 is provided with a second through-hole 117. The first and second through-holes 116 and 117 penetrate in a thickness direction (in a direction perpendicular to the plane of Fig. 9) .

The first and second connection portions 114 and 115 are caught with each other when the struts 110 are separated from each other, so that connection between the struts 110 is maintained. For this reason, compared to a case where only the polymer portion 520 is provided, it is possible to more easily maintain a strength of the stent.

As illustrated in Fig. 10, the polymer portion 520 is formed in a gap between the strut 110 and the first connection portion 114, a gap between the first connection portion 114 and the second connection portion 115, and a gap between the second connection portion 115 and the strut 110. The polymer portions 520 formed in these gaps have curved portions 521 that are concave toward the outer side from the inner side of the stent. In addition, the polymer portions 520 are also formed in the first and second through-holes 116 and 117 and also have the curved portions 521. By virtue of the curved portions 521, it is possible to obtain the same functional effects as those of the curved portions 121 of the aforementioned embodiment.

The surfaces of the first and second connection portions 114 and 115 are covered by the polymer layer 530. The polymer layer 530 and the polymer portion 520 are formed integrally with each other. The first and second connection portions 114 and 115 are bonded to and supported by the polymer layer 530 and the polymer portion 520. Therefore, the first and second connection portions 114 and 115 are not easily removed.

This application is based upon and claims the benefit of priority from the prior Japanese Patent Application No. 2015-039349 filed in Japan on February 27, 2015; the entire contents of which are incorporated herein by reference.

### [Description of Reference Numerals and Signs]

100 stent,
110 strut (metal portion),
111 gap,
113 connection portion,
114 first connection portion,
115 second connection portion,
120, 220, 320, 420, 520polymer portion,
121, 221, 321, 421, 521curved portion,
122 polymer solution,
130, 140, 530 polymer layer,
D1 axial direction of stent,
D2 radial direction of tubular outer periphery of stent,
D3 separation direction between struts (separation direction between metal portions),
L1, L2 two boundary lines between polymer portion and struts (metal portions),
L3 line obtained by straightly connecting two cross points,
P1, P4 peak of curved portion,
P2, P3 two cross points between two boundary lines and contour line of curved portion.

## Claims

1. A stent comprising:
metal portions that shape a tubular outer periphery provided with a gap; and
a polymer portion that connects the metal portions to each other in the gap,
wherein the polymer portion has a curved portion that is curved to be concave toward an outer side from an inner side in a radial direction of the tubular outer periphery.

2. The stent according to claim 1, wherein a peak where the curved portion is deepest is positioned in a center between two boundary lines formed between the polymer portion and both adjacent metal portions as seen in a cross section taken along a separation direction between the metal portions adjacent to the polymer portion.

3. The stent according to claim 1, wherein a peak where the curved portion is deepest is positioned in a location different from a center between two boundary lines formed between the polymer portion and both adjacent metal portions as seen in a cross section taken along a separation direction between the metal portions adjacent to the polymer portion.

4. The stent according to any one of claims 1 to 3, wherein a peak where the curved portion is deepest is positioned in a location different from a line obtained by straightly connecting two cross points between a contour line of the curved portion and two boundary lines formed between the polymer portion and both adjacent metal portions as seen in a cross section taken along a separation direction between the metal portions adjacent to the polymer portion.

5. The stent according to any one of claims 1 to 4, further comprising a plurality of the polymer portions and polymer layers formed on surfaces of the metal portions,
wherein the polymer layers extend along the metal portions and connect one of the polymer portions and at least one of the other polymer portions.

6. A method of manufacturing a stent, comprising:
a polymer placement process for placing polymer toward a gap formed by metal portions that shape a tubular outer periphery of a stent; and
a heating process for heating the polymer after the polymer placement process,
wherein, in the heating process, the polymer is molten by the heating and flows to the gap, so that a curved portion that is curved to be concave toward an outer side from an inner side in a radial direction of the tubular outer periphery is formed in the polymer.
